(19)

**Europäisches Patentamt**

**European Patent Office**

**Office européen des brevets**

(11) **EP 4 014 959 A1**

(12) **EUROPEAN PATENT APPLICATION**

(43) Date of publication:
**22.06.2022 Bulletin 2022/25**

(21) Application number: **20214966.2**

(22) Date of filing: **17.12.2020**

(51) International Patent Classification (IPC):
$A61K\ 8/92^{(2006.01)}$ $A61Q\ 5/06^{(2006.01)}$
$A61K\ 8/37^{(2006.01)}$ $A61K\ 8/02^{(2006.01)}$
$A61K\ 8/49^{(2006.01)}$ $A61K\ 8/73^{(2006.01)}$
$A61K\ 8/06^{(2006.01)}$

(52) Cooperative Patent Classification (CPC):
**A61K 8/92; A61K 8/0216; A61K 8/062;
A61K 8/375; A61K 8/4993; A61K 8/731;
A61K 8/922; A61Q 5/06**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Kao Corporation
Chuo-ku,
Tokyo 103-8210 (JP)**

(72) Inventors:
• **Schmid, Sabine
64297 Darmstadt (DE)**
• **Klutzny, Jutta
64297 Darmstadt (DE)**

(74) Representative: **Hoffmann Eitle
Patent- und Rechtsanwälte PartmbB
Arabellastraße 30
81925 München (DE)**

(54) **SOLID HAIR STYLING COMPOSITION**

(57)     The present invention is directed to a solid hair styling composition comprising a) one or more triglycerides being esterified with fatty acids having a carbon chain length in the range of 6 to 14 ($C_6$ to $C_{14}$), b) one or more triglycerides being esterified with fatty acids having a carbon chain length of 16 or more ($C_{16}$ or more) and/or one or more waxe(s), c) one or more non-ionic polymer(s) having at least one carbohydrate monomer unit(s), d) one or more non-ionic surfactant(s), wherein the weight ratio of compounds according to group a) to compounds according to group b) is more than 1.

EP 4 014 959 A1

**Description**

**Field of the invention**

The present invention is directed to a solid hair styling composition. In addition, a method for styling the hair is disclosed.

**Background of the invention**

Traditional hair styling compositions employ synthetic polymers, which confer the hair a certain degree of hold. However, the environmentally concerned customer demands polymer-free styling products or products comprising naturally derived polymers. Cosmetic industry has answered this demand by developing pastes and pomades, which comprise large amounts of fatty compounds. The disadvantage of these products from the viewpoint of hair styling is their low hold and durability, while leaving an oily film on the hair surface.

Therefore, there is an unmet consumer need for the provision of hair styling products, which are based on natural ingredients, are light to hair, and provide sufficient hold at the same time.

**Summary of the invention**

Therefore, the first object of the present invention is a solid hair styling composition comprising:

a) one or more triglycerides being esterified with fatty acids having a carbon chain length in the range of 6 to 14 ($C_6$ to $C_{14}$),

b) one or more triglycerides being esterified with fatty acids having a carbon chain length of 16 or more ($C_{16}$ or more), and/or one or more waxes, and/or their mixture(s),

c) one or more non-ionic polymer(s) having at least one carbohydrate monomer unit(s),

d) one or more non-ionic surfactant(s),

wherein the weight ratio of compounds according to group a) to compounds according to group b) is more than 1.

The second object of the present invention is a method for styling the hair, preferably human hair, comprising the steps of:

a) optionally washing and shampooing the hair followed with optional drying of the hair,

b) contacting the composition as defined above with the user's hand for a time period in the range of 5 s to 2 min and partially or fully allowing it to melt,

c) distributing the composition in the hair with the user's hand,

d) optionally blow-drying the hair.

**Detailed description of the invention**

Inventors of the present invention have unexpectedly found out, that a composition according to claim 1 provides good hold, low oily feel and does not weigh down the hair.

**Hair styling product**

The present invention is directed to a solid hair styling composition comprising:

a) one or more triglycerides being esterified with fatty acids having a carbon chain length in the range of 6 to 14 ($C_6$ to $C_{14}$),

b) one or more triglycerides being esterified with fatty acids having a carbon chain length of 16 or more ($C_{16}$ or more), and/or one or more waxes, and/or their mixture(s),

c) one or more non-ionic polymer(s) having at least one carbohydrate monomer unit(s),

d) one or more non-ionic surfactant(s),

wherein the weight ratio of compounds according to group a) to compounds according to group b) is more than 1.

The term 'solid' within the meaning of the present invention denotes a composition, which is solid at 25°C under atmospheric pressure.

It is preferred from the viewpoint of user convenience, that the composition of the present invention is a solid oil-in-water emulsion and comprises 40% by weight or more of water, calculated to the total weight of the composition.

It is further preferred from the viewpoint of user convenience, that the composition of the present invention at 45% by weight or more, preferably it comprises water at 50% by weight or more, calculated to the total weight of the composition.

From the viewpoint of solidification, the composition of the present invention comprises water at 90% by weight or less, preferably it comprises water at 80% by weight or less, further more preferably it comprises water at 70% by weight or less, still further more preferably it comprises water at 60% by weight or less, calculated to the total weight of the composition.

For attaining the above-mentioned effects, it is preferred that the composition of the present invention comprises water in the range from 40% to 90% by weight, more preferably in the range of 45% to 80% by weight, further more preferably in the range of 50% to 70% by weight, still further more preferably in the range of 50% to 60% by weight, calculated to the total weight of the composition.

**Compound(s) according to group a)**

The composition of the present invention comprises one or more triglycerides being esterified with fatty acids having a carbon chain length in the range of 6 to 14 ($C_6$ to $C_{14}$) as compound(s) according to group a).

It is preferred from the viewpoint of biodegradability, that one or more compound(s) according to group a) is a natural or partially synthetic oil comprising triglycerides with a fraction of esterified $C_6$ to $C_{14}$ fatty acids of 50% by weight or more, preferably 60% by weight or more, more preferably 80% by weight or more, calculated to the total weight of the natural, or partially synthetic oil according to compound(s) according to group a).

Suitable natural oils according to this definition are, for example, selected from coconut oil, palm kernel oil, and/or their mixtures.

Suitable partially synthetic oils according to this definition are, for example, hydrogenated coco-glycerides, caprylic / capric triglycerides, middle chain triglycerides, and/or their mixtures.

It is preferred from the viewpoint of commercial availability, that one or more compound(s) according to group a) is coconut oil and/or hydrogenated coco glycerides, and/or their mixtures.

It is preferred from the viewpoint of hair hold, that the total concentration of compound(s) according to group a) is 10% by weight or more, preferably 12% by weight or more, further more preferably 15% by weight or more, still more preferably 20% by weight or more, calculated to the total weight of the composition.

It is further preferred from the viewpoint of oily feel, that the total concentration of compound(s) according to group a) is 50% by weight or less, preferably 40% by weight or less, further more preferably 30% by weight or less, calculated to the total weight of the composition.

For attaining the above-mentioned effects, it is preferred, that the total concentration of compound(s) according to group a) is in the range of 10% to 50% by weight, more preferably 12% to 40% by weight, further more preferably in the range of 15% to 30% by weight, still more preferably 20% to 30% by weight, calculated to the total weight of the composition.

**Compound(s) according to group b)**

The present invention comprises one or more triglycerides being esterified with fatty acids having a carbon chain length of 16 or more ($C_{16}$ or more), and/or one or more waxes, and/or their mixture(s), according to group b).

It is preferred from the viewpoint of conditioning effect that the one or more triglycerides being esterified with fatty acids having a carbon chain length in the range of 16 or more and 40 or less, preferably in the range of 16 or more and 32 or less, more preferably in the range of 16 or more and 22 or less.

Suitable compounds according to this definition are, for example, natural oils being solid at 25°C and atmospheric pressure. It is preferred from the viewpoint of commercial availability, that one or more compound(s) according to group b) is/are selected from shea butter, avocado butter, mango butter, cocoa butter, hydrogenated vegetable oil, and/or their mixtures, further more preferably it is shea butter.

Further suitable compounds according to this definition are, for example, natural waxes. It is preferred from the viewpoint of commercial availability, that one or more compound(s) according to group b) is/are selected from carnauba wax, bees wax, candellila wax, jojoba oil, rice bran wax, soy wax, and/or their mixtures, more preferably it is carnauba wax.

It is to be noted, that mixtures of natural waxes and natural oils being solid at 25°C and atmospheric pressure may be employed as well. A preferred mixture is hydrogenated vegetable oil and carnauba wax, preferably in a weight ratio of hydrogenated vegetable oil to carnauba wax in the range of 1 to 2.

It is preferred from the viewpoint of solidification of the composition that the total concentration of compound(s) according to group b) is 5% by weight or more, preferably 8% by weight or more, further more preferably 10% by weight or more, still more preferably 15% by weight or more, calculated to the total weight of the composition.

It is preferred from the viewpoint of melting behavior that the total concentration of compound(s) according to group b) is 50% by weight or less, preferably 40% by weight or less, further more preferably 30% by weight or less, calculated to the total weight of the composition.

For attaining the above-mentioned effects, it is preferred that the total concentration of compound(s) according to group b) is in the range of 5% to 50% by weight, preferably in the range of 8% to 40% by weight, further more preferably in the range of 10% to 30% by weight, still more preferably in the range of 15% to 30% by weight, calculated to the total weight of the composition.

It is further preferred from the viewpoint of melting behavior the weight ratio of compounds according to group a) to compounds according to group b) is 1.0 or more, preferably 1.2 or more, further more preferably it is 1.5 or more.

It is preferred from the viewpoint of solidification that the weight ratio of compounds according to group a) to compounds according to group b) is 3.0 or less, preferably 2.5 or less, more preferably 2.0 or less.

For attaining the above-mentioned effects, it is preferred that the weight ratio of compounds according to group a) to compounds according to group b) is in the range of 1.0 to 3.0, preferably in the range of 1.2 to 2.5, more preferably in the range of 1.5 to 2.0.

**Compound(s) according to group c)**

The composition of the present invention comprises one or more non-ionic polymer(s) having at least one carbohydrate monomer unit(s) as compound(s) according to group c).

Suitable compound(s) according to group c) may be selected from cellulose, preferably cellulose fibers, chemically modified cellulose fibers, lignin fibers, pectin fibers, natural starch(es), chemically modified starch(es), hydrolyzed natural starch(es), and/or their mixtures, preferably it is one or more hydrolyzed natural starch(es).

Suitable starches are rice starch, wheat starch, corn starch, potato starch, and tapioca starch, and/or their mixtures. The most preferred compound(s) according to group c) is/are corn starch and hydrolyzed corn starch, from the viewpoint of melting behavior.

It is preferred from the viewpoint of melting behavior that the total concentration of compound(s) according to c) is 2% by weight or more, more preferably 3% by weight or more, further more preferably 4% by weight or more, calculated to the total of the composition.

It is preferred from the viewpoint of solidification that the total concentration of compound(s) according to group c) is 15% by weight or less, more preferably 12% by weight or less, further more preferably 10% by weight or less, calculated to the total weight of the composition.

For attaining the above-mentioned effects, it is preferred that the concentration of compound(s) according to group c) is in the range of 2% to 15% by weight, preferably 3% to 12% by weight, more preferably 4% to 10% by weight, calculated to the total weight of the composition.

**Compound(s) according to group d)**

The composition of the present invention comprises one or more non-ionic surfactant(s) as compound(s) according to group d).

Suitable compound(s) according to group d) preferably have an HLB value in the range of 14 to 17, from the viewpoint of emulsification performance.

HLB value may be calculated according to Griffin's method by using the following equation:

$$HLB = 20 \cdot \left(1 - \frac{M_l}{M}\right)$$

While $M_l$ is the molar mass of the lipophilic part of the surfactant and M is the molar mass of the total surfactant molecule.

It is further preferred from the viewpoint of emulsification performance that one or more compound(s) according to d) is/are sorbitan ester(s), preferably PEGylated sorbitan ester(s), and/or their mixtures.

Suitable compounds are known under the trade name Polysorbate or Tween, for example Polysorbate 20, Polysorbate 40, Polysorbate 60, and Polysorbate 80.

It is preferred from the viewpoint of wettability that the total concentration of compound(s) according to group d) is 3% by weight or more, more preferably 4% by weight or more, further more preferably 5% by weight or more, calculated to the total weight of the composition.

It is preferred from the viewpoint of solidification that the total concentration of compound(s) according to group d) is 20% by weight or less, preferably 15% by weight or less, further more preferably 10% by weight or less, calculated to the total weight of the composition.

For attaining the above-mentioned effects, it is preferred that the total concentration of compound(s) according to group d) is in the range of 3% to 20% by weight, preferably in the range of 4% to 15% by weight, further more preferably in the range of 5% to 10% by weight, calculated to the total weight of the composition.

**Optional features**

It is further preferred from the viewpoint of biodegradability that the composition of the present invention is free from synthetic polymers. 'Synthetic' within the meaning of the present invention denotes polymers, which are completely manufactured by chemical synthesis.

Typically, such polymers comprise at least one monomer unit of vinylpyrrolidone, acrylate, methylacrylate, and silicone.

It is therefore preferred from the viewpoint of biodegradability that the composition of the present invention is free from polymers having monomer units of vinylpyrrolidone, acrylate, methylacrylate, and silicone.

**Method for styling hair**

The present invention is also directed to a method for styling the hair, preferably human hair, comprising the steps of:

a) optionally washing and shampooing the hair followed with optional drying of the hair,

b) contacting the composition as defined above with the user's hand for a time period in the range of 5 s to 2 min and partially or fully allowing it to melt,

c) distributing the composition in the hair with the user's hand,

d) optionally blow-drying the hair.

It is preferred from the viewpoint of styling performance in step b) that the user contacts the composition for a time period in the range of 10 s to 100 s. During this time period, a certain amount of the composition melts in the hand of the user and can then be applied to the hair in step c).

In step c), the user then styles the hair according to his/her taste.

The blow-drying step d) is optional and may be executed with warm air having a temperature in the range of 45°C to 100°C.

The following examples are to illustrate the invention, but not to limit it.

**EXAMPLES**

**Example 1**

The ingredients of the composition below were heated to 80°C and then 25 g each were poured into a silicone mold having the following dimensions: 50 mm length, 30 mm width, 20 mm height. The compositions were allowed to cool overnight.

| Ingredients | | Inv comp. 1 | Inv. Comp 2 | Inv. Comp 3 |
|---|---|---|---|---|
| | | % by weight | | |
| a) | Hydrogenated coco glyceride | 5.0 | - | 10.0 |
| | Coco nucifera oil | 7.0 | - | - |
| | Palm seed oil | - | 15.0 | 10.0 |
| b) | Hydrogenated vegetable oil | 6.0 | 10.0 | - |
| | Carnauba wax | 4.0 | - | - |
| | Shea butter | - | - | 15.0 |
| c) | Hydrolyzed corn starch | 5.0 | 5.0 | 5.0 |
| | Cellulose | 1.0 | - | - |
| d) | Polysorbate-20 | 8.0 | - | 10.0 |
| | Polysorbate-60 | - | 5.0 | - |
| - | Water | Ad 100.0 | | |
| | | | | |
| - | Weight ratio a) / b) | 1.2 | 1.5 | 1.3 |

The "in vivo" hairdresser sensory evaluation is a well-established method used since about 50 years in test salons. This type of sensory evaluation is an industry standard method for testing the properties of professional hair care products on hair. The hairdresser-experts are specialized in specific product segments e.g. color, perm, styling and care products to ensure the reliability and reproducibility of the tests.

0.5 g of the above mentioned compositions were applied onto freshly cleansed dry hair. It was observed that the styled hair had good hold and durability, while appearing natural, and leaving no oiliness.

**Claims**

1.  A solid hair styling composition comprising:

    a) one or more triglycerides being esterified with fatty acids having a carbon chain length in the range of 6 to 14 ($C_6$ to $C_{14}$),
    b) one or more triglycerides being esterified with fatty acids having a carbon chain length of 16 or more ($C_{16}$ or more), and/or one or more waxes, and/or their mixture(s),
    c) one or more non-ionic polymer(s) having at least one carbohydrate monomer unit(s),
    d) one or more non-ionic surfactant(s),

    wherein the weight ratio of compounds according to group a) to compounds according to group b) is more than 1.

2.  The composition according to claim 1 **characterized in that** one or more compound(s) according to group a) is a natural or partially synthetic oil comprising triglycerides with a fraction of esterified $C_6$ to $C_{14}$ fatty acids of 50% by weight or more, preferably 60% by weight or more, more preferably 80% by weight or more, calculated to the total weight of the natural, or partially synthetic oil according to compound(s) of group a).

3.  The composition according to any of the claims 1 and/or 2 **characterized in that** one or more compound(s) according to group a) is selected from coconut oil, palm kernel oil, hydrogenated coco-glycerides, caprylic /capric triglycerides, middle chain triglycerides, and/or their mixtures, preferably it is coconut oil and/or hydrogenated coco glycerides, and/or their mixtures.

4.  The composition according to any of the preceding claims **characterized in that** the total concentration of compound(s) according to group a) is 10% by weight or more, preferably 12% by weight or more, further more preferably 15% by weight or more, still more preferably 20% by weight or more, calculated to the total weight of the composition.

5. The composition according to any of the preceding claims **characterized in that** one or more compound(s) according to group b) is/are natural wax(es), preferably selected from carnauba wax, bees wax, candellila wax, jojoba oil, rice bran wax, soy wax, and/or their mixtures, preferably it is carnauba wax.

6. The composition according to any of the preceding claims **characterized in that** one or more compound(s) according to group b) is a natural oil being solid at 25°C and atmospheric pressure, preferably selected from shea butter, avocado butter, mango butter, cocoa butter, hydrogenated vegetable oil, and/or their mixtures, more preferably it is shea butter.

7. The composition according to any of the preceding claims **characterized in that** the total concentration of compound(s) according to group b) is 5% by weight or more, preferably 8% by weight or more, further more preferably 10% by weight or more, still more preferably 15% by weight or more, calculated to the total weight of the composition.

8. The composition according to any of the preceding claims **characterized in that** the weight ratio of compounds according to group a) to compounds according to group b) is 1.0 or more, preferably 1.2 or more, further more preferably it is 1.5 or more.

9. The composition according to any of the preceding claims **characterized in that** one or more compound(s) according to group c) is selected from cellulose, preferably cellulose fibers, chemically modified cellulose fibers, lignin fibers, pectin fibers, natural starch(es), chemically modified starch(es), hydrolyzed natural starch(es), and/or their mixtures, preferably it is one or more hydrolyzed natural starch(es).

10. The composition according to any of the preceding claims **characterized in that** the concentration of compound(s) according to group c) is in the range of 2% to 15% by weight, preferably 3% to 12% by weight, more preferably 4% to 10% by weight, calculated to the total weight of the composition.

11. The composition according to any of the preceding claims **characterized in that** one or more compound(s) according to group d) has an HLB value in the range of 14 to 17.

12. The composition according to any of the preceding claims **characterized in that** one or more compound(s) according to group d) is/are sorbitan ester(s), preferably PEGylated sorbitan ester(s), and/or their mixtures.

13. The composition according to any of the preceding claims **characterized in that** the total concentration of compound(s) according to group d) is in the range of 3% to 20% by weight, preferably in the range of 4% to 15% by weight, further more preferably in the range of 5% to 10% by weight, calculated to the total weight of the composition.

14. The composition according to any of the preceding claims **characterized in that** it is a solid oil-in-water emulsion and comprises 40% by weight or more of water, calculated to the total weight of the composition.

15. A method for styling the hair, preferably human hair, comprising the steps of:

   a) optionally washing and shampooing the hair followed with optional drying of the hair,
   b) contacting the composition as defined in the claims 1 to 14 with the user's hand for a time period in the range of 5 s to 2 min and partially or fully allowing it to melt,
   c) distributing the composition in the hair with the user's hand,
   d) optionally blow-drying the hair.

Europäisches
Patentamt

European
Patent Office

Office européen
des brevets

# EUROPEAN SEARCH REPORT

Application Number

EP 20 21 4966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/215300 A1 (BEIERSDORF DAILY CHEMICAL WUHAN CO LTD [CN]; BEIERSDORF AG [DE]) 29 October 2020 (2020-10-29) * page 10, paragraphs 3,4; claims 1,7,9; examples 1,7; table 1 * | 1-15 | INV. A61K8/92 A61Q5/06 A61K8/37 A61K8/02 A61K8/49 |
| A | US 2019/029948 A1 (AKERELE DOMINIC [US] ET AL) 31 January 2019 (2019-01-31) * examples 1,2 * | 1-15 | A61K8/73 A61K8/06 |
| X | DATABASE GNPD [Online] MINTEL; 25 April 2018 (2018-04-25), anonymous: "Hair Styling Stick", XP055814633, Database accession no. 5627757 * abstract * | 1-15 | |

TECHNICAL FIELDS
SEARCHED (IPC)

A61K
A61Q

The present search report has been drawn up for all claims

| Place of search | Date of completion of the search | Examiner |
|---|---|---|
| Munich | 6 July 2021 | Verrucci, Marinella |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&: member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 21 4966

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2021

| Patent document cited in search report | | Publication date | Patent family member(s) | | Publication date |
|---|---|---|---|---|---|
| WO 2020215300 | A1 | 29-10-2020 | WO<br>WO | 2020215300 A1<br>2020216641 A1 | 29-10-2020<br>29-10-2020 |
| US 2019029948 | A1 | 31-01-2019 | NONE | | |

EPO FORM P0459

For more details about this annex : see Official Journal of the European Patent Office, No. 12/82